# EUROPEAN PATENT APPLICATION

(11) **EP 4 847 026 A1**
(43) Date of publication of application: **02.12.2026**
(21) Application number: 26178832.7
(22) Date of filing: 14.05.2026
(51) Int. Cl.: A61K 31/7004, A61K 33/06, A61K 33/20, A61K 33/24, A61M 1/28, A61P 7/08

(54) **PERITONEAL DIALYSATE, PERITONEAL DIALYSATE SET, COMPOSITION USED FOR PERITONEAL DIALYSIS, AND METHOD FOR PERFORMING PERITONEAL DIALYSIS**

(30) Priority: 29.05.2025 JP 2025089505
(71) Applicant: Terumo Kabushiki Kaisha, Tokyo 151-0072 (JP)
(72) Inventor: TAKAHASHI, Hiroaki, Kanagawa, 259-0151 (JP)
(74) Representative: KIPA AB

(57) **Abstract**

Provided is a means capable of appropriately managing water balance while enhancing the total amount of sodium removed in incremental peritoneal dialysis. A peritoneal dialysate contains 1.5 to 1.8 w/v% of glucose and 120 to 130 mEq/L of sodium, the peritoneal dialysate is used in combination with another peritoneal dialysate containing 1.35 to 1.5 w/v% of glucose and 132 to 135 mEq/L of sodium, the peritoneal dialysate and the another peritoneal dialysate are each used alone for peritoneal dialysis in which an amount of 1.5 to 2.0 L is injected into, stored in, and drained from an abdominal cavity of a subject, for a total of twice per day, in which the peritoneal dialysate is used once per day, and the another peritoneal dialysate is used once per day.

## Description

### TECHNICAL FIELD

The present invention relates to a peritoneal dialysate, a peritoneal dialysate set, a composition used for peritoneal dialysis, and a method for performing peritoneal dialysis.

### BACKGROUND ART

Stepwise peritoneal dialysis (incremental peritoneal dialysis) is a method that depends on residual renal function, and is a method in which a small number of bag exchanges of peritoneal dialysate are performed at the initial stage of introduction when the residual renal function is maintained. According to Nakayama M. Why should we use a low sodium dialysis solution for peritoneal dialysis? Perit Dial Int. 2024 Mar; 44(2): 89-97., the sodium concentrating ability of the kidneys in dialysis patients is reduced, and the urinary sodium concentration is around 70 mEq/L regardless of urine volume, so that there is a limitation on the urinary sodium excretion amount in incremental peritoneal dialysis. When compared per amount of water removed, the amount of sodium removed by peritoneal dialysis is greater than the amount of sodium removed by the residual kidney. However, when bag exchanges of peritoneal dialysate are performed twice, the total amount of sodium removed by both together cannot be said to be sufficient. For this reason, sodium may gradually accumulate in the body, potentially leading to fluid overload, which is a clinical issue.

WO 2019/168063 discloses a means capable of appropriately managing the balance between water and sodium while avoiding adverse effects on residual renal function without causing hyponatremia. However, this is limited to common peritoneal dialysis prescribed for 3 to 5 exchanges per day, and WO 2019/168063 does not disclose any solution to the problem in peritoneal dialysis prescribed for two exchanges per day that depends on residual renal function.

Depending on the degree of enhancement of the amount of sodium removed by peritoneal dialysis, it is considered that there is a possibility that a compensatory reaction of residual functional nephrons occurs to affect the urinary sodium excretion amount, and as a result, the total amount of sodium removed is not improved, and there is also a possibility that the urine volume is changed to worsen water balance, and the like.

There are many unclear points, and there is no research report that has so far clarified the effect on the urinary sodium excretion amount when the amount of sodium removed by peritoneal dialysis is enhanced at the initial stage of introduction when residual renal function is maintained.

### SUMMARY OF INVENTION

Therefore, an object of the present invention is to provide a means capable of appropriately managing water balance while enhancing the total amount of sodium removed in incremental peritoneal dialysis.

The present inventor has conducted extensive studies to solve the above problems. As a result, the present inventor has found that the above problems can be solved by using a peritoneal dialysate containing 1.5 to 1.8 w/v% of glucose and 120 to 130 mEq/L of sodium in combination with a peritoneal dialysate containing 1.35 to 1.5 w/v% of glucose and 132 to 135 mEq/L of sodium, and has completed the present invention.

In other words, the above problems can be solved by the present invention having the following configuration, and the present invention includes the following aspects and embodiments.

One aspect of the present invention is
(1) a peritoneal dialysate containing 1.5 to 1.8 w/v% of glucose and 120 to 130 mEq/L of sodium, wherein
   the peritoneal dialysate is used in combination with another peritoneal dialysate containing 1.35 to 1.5 w/v% of glucose and 132 to 135 mEq/L of sodium, and
   the peritoneal dialysate and the another peritoneal dialysate are each used alone for peritoneal dialysis in which an amount of 1.5 to 2.0 L is injected into, stored in, and drained from an abdominal cavity of a subject, for a total of twice per day, in which the peritoneal dialysate is used once per day, and the another peritoneal dialysate is used once per day;
(2) the peritoneal dialysate according to (1), preferably containing 123 to 128 mEq/L of sodium;
(3) the peritoneal dialysate according to (1) or (2), preferably further containing 2.0 to 4.0 mEq/L of calcium; 0.5 to 1.5 mEq/L of magnesium; 86.0 to 98.0 mEq/L of chloride; and 35 to 40 mEq/L of an alkalizing agent; and
(4) the peritoneal dialysate according to any one of (1) to (3), wherein the another peritoneal dialysate preferably contains 2.3 to 4.0 mEq/L of calcium; 0.5 to 1.5 mEq/L of magnesium; 95.0 to 105.5 mEq/L of chloride; and 35 to 40 mEq/L of an alkalizing agent.

Another aspect of the present invention is
(5) a peritoneal dialysate set including:
a first peritoneal dialysate that contains 1.5 to 1.8 w/v% of glucose and 120 to 130 mEq/L of sodium; and
a second peritoneal dialysate that contains 1.35 to 1.5 w/v% of glucose and 132 to 135 mEq/L of sodium, wherein
the first peritoneal dialysate and the second peritoneal dialysate are each used alone for peritoneal dialysis in which an amount of 1.5 to 2.0 L is injected into, stored in, and drained from an abdominal cavity of a subject, for a total of twice per day, in which the first peritoneal dialysate is used once per day, and the second peritoneal dialysate is used once per day.

Another aspect of the present invention is
(6) a composition for use in peritoneal dialysis,
wherein the composition contains 1.5 to 1.8 w/v% of glucose and 120 to 130 mEq/L of sodium,
wherein the composition is used in combination with another composition for use in peritoneal dialysis containing 1.35 to 1.5 w/v% of glucose and 132 to 135 mEq/L of sodium,
wherein the composition and the another composition are independently injected into, stored in, and drained from an abdominal cavity of a subject in an amount of 1.5 to 2.0 L, for a total of twice per day, in which the composition is used once per day, and the another composition is used once per day.

Another aspect of the present invention is
(7) a method for performing peritoneal dialysis on a single subject in a single day, the method including administering a first peritoneal dialysate to the single subject once in the single day and administering a second peritoneal dialysate to the single subject once in the single day, wherein
the first peritoneal dialysate contains 1.5 to 1.8 w/v% of glucose and 120 to 130 mEq/L of sodium,
the second peritoneal dialysate contains 1.35 to 1.5 w/v% of glucose and 132 to 135 mEq/L of sodium,
the administering of the first peritoneal dialysate includes injecting 1.5 to 2.0 L of the first peritoneal dialysate into an abdominal cavity of the single subject, storing the first peritoneal dialysate in the abdominal cavity, and draining the first peritoneal dialysate from the abdominal cavity,
the administering of the second peritoneal dialysate includes injecting 1.5 to 2.0 L of the second peritoneal dialysate into the abdominal cavity of the single subject, storing the second peritoneal dialysate in the abdominal cavity, and draining the second peritoneal dialysate from the abdominal cavity, and
the injecting of the first peritoneal dialysate and the injecting of the second peritoneal dialysate are independent of each other.

### DESCRIPTION OF EMBODIMENTS

Hereinafter, an embodiment according to one mode of the present invention will be described. The present invention is not limited only to the following embodiment.

In the present description, "X to Y" indicating a range means "X or more and Y or less". Further, unless otherwise specified, operations and measurements of physical properties and the like are performed under conditions of room temperature (20 to 25°C)/relative humidity of 40 to 50% RH.

### <Peritoneal Dialysate>

One aspect of the present invention is a peritoneal dialysate containing 1.5 to 1.8 w/v% of glucose and 120 to 130 mEq/L of sodium, wherein
the peritoneal dialysate is used in combination with another peritoneal dialysate containing 1.35 to 1.5 w/v% of glucose and 132 to 135 mEq/L of sodium, and
the peritoneal dialysate and the another peritoneal dialysate are each used alone for peritoneal dialysis in which an amount of 1.5 to 2.0 L is injected into, stored in, and drained from the abdominal cavity of a subject, for a total of twice per day, in which the peritoneal dialysate is used once per day, and the another peritoneal dialysate is used once per day. With such a configuration, it is possible to appropriately manage water balance while enhancing the total amount of sodium removed in incremental peritoneal dialysis.

The peritoneal dialysate according to the present aspect (also referred to herein as the "first peritoneal dialysate") contains 1.5 to 1.8 w/v% of glucose and 120 to 130 mEq/L of sodium. The first peritoneal dialysate preferably contains 123 to 128 mEq/L of sodium, more preferably 125 to 128 mEq/L of sodium. In one embodiment, the first peritoneal dialysate contains 1.6 w/v% of glucose and 126 mEq/L of sodium.

The first peritoneal dialysate can further contain components contained in normal peritoneal dialysates. Examples of such components include calcium, magnesium, chloride, alkalinizing agent, and the like. The first peritoneal dialysate preferably contains 2.0 to 4.0 mEq/L of calcium; 0.5 to 1.5 mEq/L of magnesium; 86.0 to 98.0 mEq/L of chloride; and 35 to 40 mEq/L of an alkalinizing agent.

Examples of the alkalinizing agent include lactate ions, bicarbonate ions, and the like. The concentration of the alkalinizing agent is the sum of lactate ions, bicarbonate ions, and the like to be used, and for example, 35 to 40 mEq/L of an alkalinizing agent can be obtained by combining lactate ions and bicarbonate ions. The alkalinizing agent is preferably lactate ions.

The first peritoneal dialysate is used in combination with the another peritoneal dialysate (also referred to herein as the "second peritoneal dialysate") containing 1.35 to 1.5 w/v% of glucose and 132 to 135 mEq/L of sodium. In one embodiment, the second peritoneal dialysate contains 1.35 w/v% of glucose and 135 mEq/L of sodium.

The second peritoneal dialysate can further contain components contained in normal peritoneal dialysates. Examples of such components include calcium, magnesium, chloride, alkalinizing agent, and the like. The second peritoneal dialysate preferably contains 2.3 to 4.0 mEq/L of calcium; 0.5 to 1.5 mEq/L of magnesium; 95.0 to 105.5 mEq/L of chloride; and 35 to 40 mEq/L of an alkalinizing agent.

As the alkalinizing agent contained in the second peritoneal dialysate, the same alkalinizing agent as the alkalinizing agent contained in the first peritoneal dialysate described above can be used.

The first peritoneal dialysate and the second peritoneal dialysate can further contain other components as necessary as long as the object of the present invention is not impaired. For example, the first peritoneal dialysate and the second peritoneal dialysate can contain an organic acid or the like depending on the difference in concentration between total cations and chloride ions in order to maintain the electrical neutrality of the peritoneal dialysate. Examples of such an organic acid include propionic acid, malic acid, fumaric acid, succinic acid, oxaloacetic acid, N-acetylglycine, N-acetyl-L-cysteine, glutaric acid, glucuronic acid, ascorbic acid, citric acid, isocitric acid, gluconic acid, N-acetyl-L-aspartic acid, N-acetyl-L-glutamic acid, N-acetyl-L-methionine, N-acetyl-L-proline, N-acetyl-L-valine, N-acetyl-L-glutamine, N-acetyl-L-arginine, N-acetyl-L-histidine, N-acetyl-L-leucine, N-acetyl-L-tryptophan, salts thereof, and the like.

The pH of the first peritoneal dialysate and the second peritoneal dialysate is usually about pH 5.0 to 7.5, preferably about pH 6.5 to 7.5.

The osmotic pressure of the first peritoneal dialysate and the second peritoneal dialysate is usually about 300 to 500 mOsm/kg, preferably about 330 to 450 mOsm/kg.

In peritoneal dialysis, 1.5 to 2.0 L of peritoneal dialysate is usually injected into the abdominal cavity of the subject, stored for 4 to 8 hours, and drained and removed after an expected effect is achieved. The above operations are defined as one cycle. Incremental peritoneal dialysis is a stepwise peritoneal dialysis initiation method in which renal replacement therapy is started with a prescription for a small number of peritoneal dialysis bags at the initial stage of introduction when residual renal function is maintained, and the dialysis dose is increased according to the degree of decline in residual renal function (Peritoneal Dialysis and Kidney Transplantation Handbook, CHUGAI-IGAKUSHA, October 15, 2018). In peritoneal dialysis according to the present aspect, a total of two operations are performed per day. In the peritoneal dialysis according to the present aspect, preferably, two consecutive operations are continuously performed. The first peritoneal dialysate is used in combination with the second peritoneal dialysate. In other words, the first peritoneal dialysate is used alone once per day, and the second peritoneal dialysate is used alone once per day. In this way, by using the first peritoneal dialysate in an appropriate combination with the second peritoneal dialysate, it is possible to appropriately manage water balance while enhancing the total amount of sodium removed in incremental peritoneal dialysis.

The subject for peritoneal dialysis is, for example, a mammal, preferably a human. In one embodiment, the subject according to the present aspect is a subject who is in need of peritoneal dialysis twice per day as determined by a physician. In one embodiment, the subject according to the present aspect is a subject having an estimated glomerular filtration rate (eGFR) of 15.0 (mL/min/1.73 m²) or less. The eGFR of the subject can be 2.7 (mL/min/1.73 m²) or more. In other words, the eGFR of the subject according to the present aspect can be 2.7 (mL/min/1.73 m²) or more and 15.0 (mL/min/1.73 m²) or less, or 4.8 (mL/min/1.73 m²) or more and 7.7 (mL/min/1.73 m²) or less.

The injection volume, the retention time, and the number of operations are appropriately adjusted depending on symptoms, blood biochemical values, imbalance of body fluid, age, body weight, and the like. The injection rate and the drainage rate are usually 300 mL/min or less.

A method for preparing the first peritoneal dialysate and the second peritoneal dialysate is not particularly limited, and general methods for preparing a peritoneal dialysate can be used. For example, a method can be used in which glucose; cation and chloride ion sources such as sodium chloride, calcium chloride, magnesium chloride, sodium lactate, calcium salts, magnesium salts, and sodium bicarbonate; an acid component; and the like are dissolved in water (e.g., water for injection) such that the concentration of glucose and the concentration of sodium, and if necessary, the concentrations of calcium, magnesium, chloride, an alkalinizing agent, and the like fall within the above ranges.

The prepared peritoneal dialysate is preferably sealed in a soft plastic bag, a glass container, or the like, and then subjected to high-pressure steam sterilization or hot water sterilization. Examples of the material of the soft plastic include polyvinyl chloride, polypropylene, polyethylene, polyester, polyamide, ethylene vinyl alcohol copolymer, polyethylene terephthalate, polyvinylidene chloride, ethylene vinyl acetate copolymer, and the like, and these may be laminated and combined.

The peritoneal dialysate according to the present aspect includes the following embodiment:
A peritoneal dialysate containing 1.5 to 1.8 w/v% of glucose and 120 to 130 mEq/L of sodium, wherein
the peritoneal dialysate is used in combination with another peritoneal dialysate containing 1.35 to 1.5 w/v% of glucose and 132 to 135 mEq/L of sodium.

### <Peritoneal Dialysate Set>

Another aspect of the present invention is a peritoneal dialysate set including:
a first peritoneal dialysate that contains 1.5 to 1.8 w/v% of glucose and 120 to 130 mEq/L of sodium; and
a second peritoneal dialysate that contains 1.35 to 1.5 w/v% of glucose and 132 to 135 mEq/L of sodium, wherein
the first peritoneal dialysate and the second peritoneal dialysate are each used alone for peritoneal dialysis in which an amount of 1.5 to 2.0 L is injected into, stored in, and drained from the abdominal cavity of a subject, for a total of twice per day, in which the first peritoneal dialysate is used once per day, and the second peritoneal dialysate is used once per day. With such a configuration, it is possible to appropriately manage water balance while enhancing the total amount of sodium removed in incremental peritoneal dialysis.

In the peritoneal dialysate set according to the present aspect, the descriptions of the "first peritoneal dialysate", the "second peritoneal dialysate", and the "peritoneal dialysis (peritoneal dialysis solution)" are the same as those of the peritoneal dialysate according to the present aspect described above, and thus the descriptions thereof are omitted.

The peritoneal dialysate set according to the present aspect includes the following embodiments:
A peritoneal dialysate set including:
a first peritoneal dialysate that contains 1.5 to 1.8 w/v% of glucose and 120 to 130 mEq/L of sodium; and
a second peritoneal dialysate that contains 1.35 to 1.5 w/v% of glucose and 132 to 135 mEq/L of sodium, wherein
the first peritoneal dialysate and the second peritoneal dialysate are used alone;

A peritoneal dialysate set to be used in a day, including:
one dose of a first peritoneal dialysate that contains 1.5 to 1.8 w/v% of glucose and 120 to 130 mEq/L of sodium; and
one dose of a second peritoneal dialysate that contains 1.35 to 1.5 w/v% of glucose and 132 to 135 mEq/L of sodium, wherein
the amount of the first peritoneal dialysate is 1.5 to 2.0 L, and the amount of the second peritoneal dialysate is 1.5 to 2.0 L of use each time;

A peritoneal dialysate set for performing peritoneal dialysis on a single subject in a single day, including:
a first volume of a first peritoneal dialysate solution that contains 1.5 to 1.8 w/v% of glucose and 120 to 130 mEq/L of sodium; and
a second volume of a second peritoneal dialysate solution that contains 1.35 to 1.5 w/v% of glucose and 132 to 135 mEq/L of sodium, wherein
the sum of the first volume of the first peritoneal dialysate solution and the second volume of the second peritoneal dialysate solution is at least 3.0 L, and
the peritoneal dialysate set being constituted so that the first peritoneal dialysate solution and the second peritoneal dialysate solution are independently injected during peritoneal dialysis and so that a total volume of at least 3.0 L is injected into the abdominal cavity of the single subject in the single day; and

A peritoneal dialysate set for performing peritoneal dialysis on a single subject in a single day, including:
one container holding 1.5 to 2.0 L of a first peritoneal dialysate solution that contains 1.5 to 1.8 w/v% of glucose and 120 to 130 mEq/L of sodium;
one container holding 1.5 to 2.0 L of a second peritoneal dialysate solution that contains 1.35 to 1.5 w/v% of glucose and 132 to 135 mEq/L of sodium; and
the peritoneal dialysate set being configured so that the first peritoneal dialysate solution and the second peritoneal dialysate solution are independently injected during peritoneal dialysis, and so that a total volume of at least 3.0 L is injected into the abdominal cavity of the single subject in the single day.

### <Composition Used for Peritoneal Dialysis>

Another aspect of the present invention is a composition for use in peritoneal dialysis,
wherein the composition contains 1.5 to 1.8 w/v% of glucose and 120 to 130 mEq/L of sodium,
wherein the composition is used in combination with another composition for use in peritoneal dialysis containing 1.35 to 1.5 w/v% of glucose and 132 to 135 mEq/L of sodium,
wherein the composition and the another composition are independently injected into, stored in, and drained from an abdominal cavity of a subject in an amount of 1.5 to 2.0 L, for a total of twice per day, in which the composition is used once per day, and the another composition is used once per day.

In the composition used for peritoneal dialysis according to the present aspect, the "composition for use in peritoneal dialysis" corresponds to the "first peritoneal dialysate" of the peritoneal dialysate according to the present aspect described above, and the "another composition for use in peritoneal dialysis" corresponds to the "second peritoneal dialysate" of the peritoneal dialysate according to the present aspect described above. Therefore, the descriptions of the "composition for use in peritoneal dialysis", the "another composition for use in peritoneal dialysis", and the "peritoneal dialysis (peritoneal dialysis solution)" are the same as those of the peritoneal dialysate according to the present aspect described above, and thus the descriptions thereof are omitted.

The composition for use in peritoneal dialysis according to the present aspect includes the following embodiments:
A combination for performing peritoneal dialysis on a single subject in a single day, including:
a composition (first composition) containing 1.5 to 1.8 w/v% of glucose and 120 to 130 mEq/L of sodium; and
another composition (second composition) containing 1.35 to 1.5 w/v% of glucose and 132 to 135 mEq/L of sodium,
combination including a total volume of the first and second compositions that (i) allows the first composition to be independently injected into the abdominal cavity of the single subject in the single day in an amount of 1.5 to 2.0 L once a day, and (ii) allows the second composition to be independently injected into the abdominal cavity of the single subject in the single day in an amount of 1.5 to 2.0 L once a day.

### <Method for Performing Peritoneal Dialysis>

Another aspect of the present invention is a method for performing peritoneal dialysis on a single subject in a single day, the method including administering a first peritoneal dialysate (first peritoneal dialysate solution) to the single subject once in the single day and administering a second peritoneal dialysate (second peritoneal dialysate solution) to the single subject once in the single day, wherein
the first peritoneal dialysate contains 1.5 to 1.8 w/v% of glucose and 120 to 130 mEq/L of sodium,
the second peritoneal dialysate contains 1.35 to 1.5 w/v% of glucose and 132 to 135 mEq/L of sodium,
the administering of the first peritoneal dialysate includes injecting 1.5 to 2.0 L of the first peritoneal dialysate into the abdominal cavity of the single subject, storing the first peritoneal dialysate in the abdominal cavity, and draining the first peritoneal dialysate from the abdominal cavity,
the administering of the second peritoneal dialysate includes injecting 1.5 to 2.0 L of the second peritoneal dialysate into the abdominal cavity of the single subject, storing the second peritoneal dialysate in the abdominal cavity, and draining the second peritoneal dialysate from the abdominal cavity, and
the injecting of the first peritoneal dialysate and the injecting of the second peritoneal dialysate are independent of each other. With such a configuration, it is possible to appropriately manage water balance while enhancing the total amount of sodium removed in incremental peritoneal dialysis.

In the method for performing peritoneal dialysis according to the present aspect, the descriptions of the "first peritoneal dialysate (first peritoneal dialysate solution)", the "second peritoneal dialysate (second peritoneal dialysate solution)", and the "peritoneal dialysis (peritoneal dialysis solution)" are the same as those of the peritoneal dialysate according to the present aspect described above, and thus the descriptions thereof are omitted.

### Examples

The effects of the present invention will be described using the following examples and comparative examples. However, the technical scope of the present invention is not limited only to the following examples. Note that in the following examples, the expression of "part" or "%" may be used, but unless otherwise specified, "part by mass" or "% by mass" is expressed. Further, unless otherwise specified, each operation is performed at room temperature (1 to 30°C).

### <Preparation of Peritoneal Dialysate>

Each required amount of anhydrous glucose, sodium chloride, calcium chloride dihydrate, magnesium chloride hexahydrate, and sodium L-lactate solution was taken so as to have each concentration of glucose, sodium, calcium, magnesium, chloride, and lactate ions shown in Table 1, and a solution was prepared in accordance with the method for producing injections as specified in the General Rules for Preparations of the Japanese Pharmacopoeia, and filtered through a membrane filter. The filtered dialysate was dispensed into and sealed in a container that is made of polypropylene resin for peritoneal perfusion and conforms to the two-chamber container test method. Thereafter, the container was hermetically sealed in a packaging bag made of a multilayer laminate film, and then sterilized by high pressure steam.

### [Table 1]

**(Table 1)**

| | First peritoneal dialysate | Second peritoneal dialysate A | Second peritoneal dialysate B |
|---|---|---|---|
| Glucose (w/v%) | 1.6 | 1.35 | 1.35 |
| Na⁺ (mEq/L) | 126 | 135 | 135 |
| Ca²⁺ (mEq/L) | 3.5 | 4 | 2.5 |
| Mg²⁺ (mEq/L) | 0.5 | 1.5 | 0.5 |
| Cl⁻ (mEq/L) | 95 | 105.5 | 98 |
| Lactate ions (mEq/L) | 35 | 35 | 40 |

### <Test Example>

The following clinical trial was conducted for patients undergoing peritoneal dialysis.

### (Main Selection Criteria)

Patients who met the following criteria and had the capacity to consent were targeted.
(1) Age: 20 years old or older (at the time of acquisition of written consent)
(2) Gender: any
(3) Patients who have stably undergone peritoneal dialysis for 3 months, and for whom a peritoneal dialysate containing 1.35 w/v% of glucose has been prescribed twice or more per day, or a peritoneal dialysate containing 1.35 w/v% of glucose and a peritoneal dialysate containing 2.5 w/v% of glucose have each been prescribed once or more
(4) Patients whose prescriptions have not been changed for 30 days or more before the consent was obtained
(5) Patients whom a physician has determined to need sodium removal based on physical findings, laboratory findings, or the like.

### (Main Exclusion Criteria)

(1) Patients concurrently undergoing hemodialysis
(2) Patients undergoing automated peritoneal dialysis
(3) Patients undergoing peritoneal dialysis therapy only once per day
(4) Patients who have achieved proper salt reduction management
(5) Patient showing signs of volume depletion
(6) Patient with persistent hypotension, having an office systolic blood pressure of less than 100 mmHg
(7) Patients with a serum sodium concentration of less than 130 mEq/L or patients with symptomatic hyponatremia.
(8) Other patients whom an investigator or a sub-investigator has determined to be inappropriate.

### (Discontinuation Criteria)

The investigator (sub-investigator) discontinued the clinical trial when the following criteria were met.
(1) When a subject, a legal representative, or the like requested discontinuation of the clinical trial
(2) When it was found after the start of the clinical trial that the subject did not meet the selection criteria
(3) When symptoms worsened, making it difficult to continue the clinical trial
(4) When an adverse event occurred, making it difficult to continue the clinical trial
(5) Other cases in which the investigator or the sub-investigator recognized the necessity for discontinuation.

### (Method)

Patients ongoing ambulatory peritoneal dialysis therapy were randomly assigned to an 8-week treatment in either a test drug group or a control drug group (test drug group:control drug group = 2:1). For peritoneal dialysis patients prescribed twice daily, who are the subject of the present invention, Table 2 shows a daily prescription breakdown for the test drug group and the control drug group.

### [Table 2]

**(Table 2)**

| Group name | Daily prescription breakdown | |
|---|---|---|
| Test drug group | First peritoneal dialysate (test drug) | Second peritoneal dialysate A or B (standard drug) |
| Control drug group | Second peritoneal dialysate A (control drug) | Second peritoneal dialysate A (standard drug) |
| | Second peritoneal dialysate B (control drug) | Second peritoneal dialysate B (standard drug) |

Each peritoneal dialysate was injected into the abdominal cavity in an amount of 1.5 to 2.0 L once, retained for 4 to 8 hours, and drained and removed after an expected effect was achieved. The above operations were defined as one cycle, and two consecutive operations were continuously performed per day.

The injection volume, the retention time, and the number of operations were appropriately increased or decreased depending on symptoms, blood biochemical values, imbalance of body fluid, age, body weight, and the like. The injection rate and the drainage rate were usually 300 mL/min or less.

The use of peritoneal dialysates other than the first peritoneal dialysate, a second peritoneal dialysate A, and a second peritoneal dialysate B was prohibited during an evaluation period. Further, dialysis therapies other than peritoneal dialysis therapy were prohibited.

### (Evaluation Items)

Using the drainage and the urine collected from the patient, the transperitoneal sodium removal amount and the amount of water removed per dialysis of the peritoneal dialysate, and the transperitoneal sodium removal amount, urinary sodium excretion amount, total amount of sodium removed, amount of water removed, urine volume, and total amount of water removed per day were calculated and evaluated by the following methods. The results are shown in Tables 3 and 4.

### [Transperitoneal sodium removal amount per dialysis of peritoneal dialysate (mEq/cycle)]

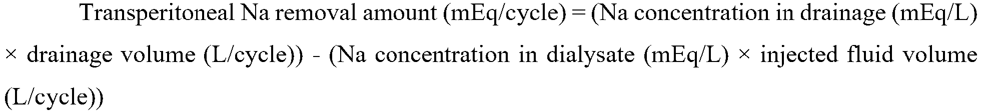

Transperitoneal Na removal amount (mEq/cycle) = (Na concentration in drainage (mEq/L) × drainage volume (L/cycle)) - (Na concentration in dialysate (mEq/L) × injected fluid volume (L/cycle))

### [Amount of water removed per dialysis of peritoneal dialysate (mL/cycle)]

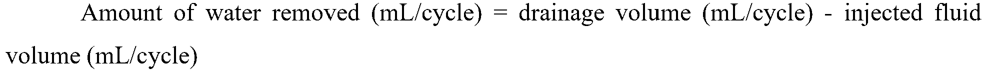

Amount of water removed (mL/cycle) = drainage volume (mL/cycle) - injected fluid volume (mL/cycle)

### [Transperitoneal sodium removal amount per day (mEq/day)]

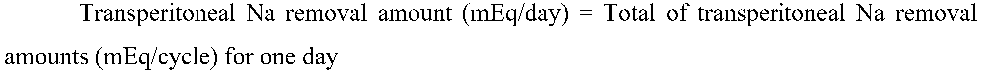

Transperitoneal Na removal amount (mEq/day) = Total of transperitoneal Na removal amounts (mEq/cycle) for one day

### [Urinary sodium excretion amount per day (mEq/day)]

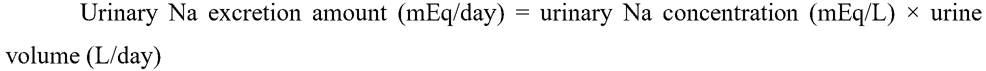

Urinary Na excretion amount (mEq/day) = urinary Na concentration (mEq/L) × urine volume (L/day)

### [Total amount of sodium removed per day (mEq/day)]

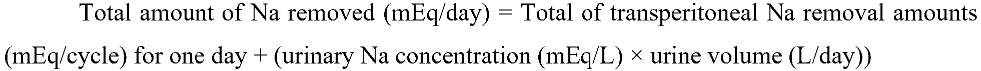

Total amount of Na removed (mEq/day) = Total of transperitoneal Na removal amounts (mEq/cycle) for one day + (urinary Na concentration (mEq/L) × urine volume (L/day))

### [Amount of water removed per day (mL/day)]

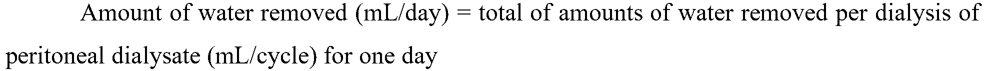

Amount of water removed (mL/day) = total of amounts of water removed per dialysis of peritoneal dialysate (mL/cycle) for one day

### [Urine volume per day (mL/day)]

$Urine volume \left(mL/day\right) = Accumulated urine \left(mL\right) for one day$

### [Total amount of water removed per day (mL/day)]

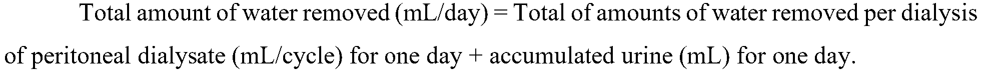

Total amount of water removed (mL/day) = Total of amounts of water removed per dialysis of peritoneal dialysate (mL/cycle) for one day + accumulated urine (mL) for one day.

In Table 3, for the test drug group, the transperitoneal sodium removal amount and the amount of water removed after peritoneal dialysis with the first peritoneal dialysate (test drug) are shown, and for the control drug group, the transperitoneal sodium removal amount and the amount of water removed after peritoneal dialysis with the second peritoneal dialysate A or B (control drug) are shown.

In Table 4, for the test drug group, the respective totals (daily amounts) of the transperitoneal sodium removal amounts, urinary sodium excretion amounts, total amounts of sodium removed, amounts of water removed, urine volumes, and total amounts of water removed after peritoneal dialysis with the first peritoneal dialysate (test drug) and the second peritoneal dialysate A or B (standard drug) are shown, and for the control drug group, the respective totals (daily amounts) of the transperitoneal sodium removal amounts, urinary sodium excretion amounts, total amounts of sodium removed, amounts of water removed, urine volumes, and total amounts of water removed after peritoneal dialysis with the second peritoneal dialysate A or B (control drug) and the second peritoneal dialysate A or B (standard drug) are shown.

### (Statistical Analysis)

The evaluation results were summarized as mean ± standard deviation, and the difference in mean between the groups and the two-sided 95% confidence interval were calculated for the amount of change from a baseline to week 8. For comparisons between the groups, an independent t-test with a two-sided significance level of 5% was used. The results are shown in Tables 3 and 4.

### [Table 3]

**(Table 3)**

| | | Test drug group | | Control drug group | | Mean between groups | |
|---|---|---|---|---|---|---|---|
| | | First peritoneal dialysate (test drug) | | Second peritoneal dialysate A or B (control drug) | | | |
| | | n | Mean ± standard deviation | n | Mean ± standard deviation | Difference (95% confidence interval) | P |
| Transperitoneal Na removal amount (mEq/time) | | | | | | | |
| | Baseline (measured value) | 18 | -2.1 ± 15.3 | 10 | 8.1 ± 19.3 | | |
| Amount of change from baseline to week 8 | | 17 | 13.8 ± 10.6 | 9 | -2.8 ± 8.1 | 16.6 (8.2, 24.9) | 0.0004 |
| Amount of water removed (mL/time) | | | | | | | |
| | Baseline (measured value) | 18 | 12.9 ± 154.1 | 9 | 53.6 ± 137.3 | | |
| Amount of change from baseline to week 8 | | 17 | 36.6 ± 85.9 | 9 | -5.8 ± 47.6 | 42.4 (-21.8, 106.5) | 0.185 |

### [Table 4]

**(Table 4)**

| | | Test drug group | | Control drug group | | Mean between groups | |
|---|---|---|---|---|---|---|---|
| | | n | Mean ± standard deviation | n | Mean ± standard deviation | Difference (95% confidence interval) | P |
| Transperitoneal Na removal amount (mEq/day) | | | | | | | |
| | Baseline (measured value) | 18 | 3.1 ± 23.5 | 10 | 16.9 ± 22.4 | | |
| Amount of change from baseline to week 8 | | 17 | 14.9 ± 15.4 | 9 | -3.6 ± 20.1 | 18.5 (4, 33) | 0.015 |
| Urinary Na excretion amount (mEq/day) | | | | | | | |
| | Baseline (measured value) | 18 | 97.4 ± 45.7 | 10 | 89.4 ± 40.1 | | |
| Amount of change from baseline to week 8 | | 17 | -7 ± 20.3 | 9 | -11.4 ± 16.3 | 4.4 (-11.8, 20.7) | 0.581 |
| Total amount of Na removed (mEq/day) | | | | | | | |
| | Baseline (measured value) | 18 | 100.6 ± 31.0 | 10 | 106.2 ± 28.9 | | |
| Amount of change from baseline to week 8 | | 17 | 8.1 ± 28.5 | 9 | -14.8 ± 18.4 | 22.9 (1.1, 44.6) | 0.04 |
| Amount of water removed (mL/day) | | | | | | | |
| | Baseline (measured value) | 18 | 84 ± 258 | 10 | 111 ± 187 | | |
| Amount of change from baseline to week 8 | | 17 | 53 ± 137 | 9 | -10 ± 146 | 63 (-57, 182) | 0.286 |
| Urine volume (mL/day) | | | | | | | |
| | Baseline (measured value) | 18 | 1371 ± 473 | 10 | 1180 ± 472 | | |
| Amount of change from baseline to week 8 | | 17 | -11 ± 242 | 9 | -21 ± 211 | 10 (-187, 208) | 0.918 |
| Total amount of water removed (mL/day) | | | | | | | |
| | Baseline (measured value) | 18 | 1455 ± 503 | 10 | 1291 ± 378 | | |
| Amount of change from baseline to week 8 | | 17 | 42 ± 314 | 9 | -31 ± 316 | 73 (-194, 341) | 0.579 |

As shown in Table 3, it can be seen that the amount of sodium removed per dialysis of the first peritoneal dialysate (test drug) was 16.6 mEq (approximately 1 g in terms of salt) more than that of the second peritoneal dialysate A or B (control drug), with a statistically significant difference. The amount of change in the amount of water removed per dialysis tended to be 42.4 mL more for the first peritoneal dialysate (test drug) than for the second peritoneal dialysate A or B (control drug), but no statistical difference was observed.

As shown in Table 4, it can be seen that in terms of the transperitoneal sodium removal amount per day, the amount of sodium removed in the test drug group was 18.5 mEq more than that in the control drug group, with a statistically significant difference. This was similar to the results of the transperitoneal sodium removal amount per dialysis of the first peritoneal dialysate (test drug). In other words, it can be seen that even when the first peritoneal dialysate (test drug) and the second peritoneal dialysate A or B are used in combination, the enhancement of the amount of sodium removed by the first peritoneal dialysate is reflected.

As shown in Table 4, in the amount of water removed per day, similar to the results per dialysis of the first peritoneal dialysate (test drug), no statistical difference was observed between the test drug group and the control drug group. In other words, it can be seen that when the first peritoneal dialysate and the second peritoneal dialysate A or B are used in combination, the amount of water removed is not affected, and only the amount of sodium removed is enhanced.

Regarding the sodium balance of the whole body, as shown in Table 4, it can be seen that there is no difference in the urinary sodium excretion amount between the test drug group and the control drug group, and the total amount of sodium removed in the test drug group is 22.9 mEq more than that in the control drug group, with a statistically significant difference. This revealed that when the first peritoneal dialysate and the second peritoneal dialysate A or B are used in combination, the total amount of sodium removed is increased without affecting urinary sodium excretion due to residual renal function.

Regarding the water balance of the whole body, as shown in Table 4, the amount of water removed, the urine volume, and the total amount of water removed were approximately the same between the test drug group and the control drug group. This revealed that when the first peritoneal dialysate and the second peritoneal dialysate A or B are used in combination, there is almost no effect on the water balance of the whole body.

From the above, it can be seen that in a patient undergoing peritoneal dialysis prescribed twice per day, by using the first peritoneal dialysate and the second peritoneal dialysate A or B in combination, the sodium balance and the water balance can be appropriately managed by enhancing only the total amount of sodium removed including peritoneal dialysis and residual renal function while maintaining the water balance of the whole body.

This application is based on Japanese Patent Application No. 2025-089505 filed on May 29, 2025, the disclosure of which is incorporated herein by reference in its entirety.

## Claims

1. A composition for use in peritoneal dialysis,
wherein the composition comprises 1.5 to 1.8 w/v% of glucose and 120 to 130 mEq/L of sodium,
wherein the composition is used in combination with another composition for use in peritoneal dialysis containing 1.35 to 1.5 w/v% of glucose and 132 to 135 mEq/L of sodium,
wherein the composition and the another composition are independently injected into, stored in, and drained from an abdominal cavity of a subject in an amount of 1.5 to 2.0 L, for a total of twice per day, in which the composition is used once per day, and the another composition is used once per day.

2. The composition for use in peritoneal dialysis according to claim 1, comprising 123 to 128 mEq/L of sodium.

3. The composition for use in peritoneal dialysis according to claim 1 or 2, further comprising 2.0 to 4.0 mEq/L of calcium; 0.5 to 1.5 mEq/L of magnesium; 86.0 to 98.0 mEq/L of chloride; and 35 to 40 mEq/L of an alkalizing agent.

4. The peritoneal dialysate according to any one of claims 1 to 3, wherein the another peritoneal dialysate further comprises 2.3 to 4.0 mEq/L of calcium; 0.5 to 1.5 mEq/L of magnesium; 95.0 to 105.5 mEq/L of chloride; and 35 to 40 mEq/L of an alkalizing agent.
